# EUROPEAN PATENT APPLICATION

(11) **EP 4 846 867 A1**
(43) Date of publication of application: **02.12.2026**
(21) Application number: 25179740.3
(22) Date of filing: 29.05.2025
(51) Int. Cl.: A24F 23/02, A61K 31/05, A61K 35/747, A61K 36/63, A61P 1/02

(54) **TOPICAL ORAL COMPOSITION**

(71) Applicant: Magisnat Omics LLC, Peachtree Corners, GA 30092 (US)
(72) Inventor: Donato, Kevin, Brescia (BS) (IT); Bertelli, Matteo, Brescia (BS) (IT)
(74) Representative: Pes, Matteo

(57) **Abstract**

It is disclosed a topical oral composition comprising *Lactobacillus reuteri* and one or more polyphenols, such as olive tree polyphenols. For example, olive tree polyphenols comprise one or more of: hydroxytyrosol, tyrosol, oleuropein. *Lactobacillus reuteri* is heat inactivated and/or tindalized. The composition may be formulated in a nicotine pouch or in a liquid formulation suitable for administration by spray, drops, rinsing, or gargling. The topical oral composition may be used as anti-inflammatory agent for oral disorders related to nicotine-containing products, as agent for restoring microbial balance following chronic nicotine exposure, for promoting and/or maintaining oral microbial homeostasis, as medicament for oral disorders related to nicotine-containing products as gingivitis, periodontitis, oral candidiasis, *Streptococcus* pyogenes-induced pharyngitis or pharyngostomatitis.

## Description

### Field of the invention

The present invention refers to a topical oral composition for preventing or treating oral disorders related to oral inflammation and/or dysbiosis, in particular oral disorders related to the use of nicotine-containing products, as for example tobacco cigarettes, electronic cigarettes, nicotine pouches.

### State of the art

It is known that smoking promotes the early acquisition and colonization of pathogens in oral biofilms. The subgingival microbial profile associated with periodontitis in smokers is diverse and distinctive compared to that of non-smokers (Biedermann L, Zeitz J, Mwinyi J, Sutter-Minder E, Rehman A, et al. (2013) Smoking Cessation Induces Profound Changes in the Composition of the Intestinal Microbiota in Humans. PLoS ONE 8(3): e59260)*.* Periodontitis and halitosis are more common in smokers and are positively correlated with lifelong smoke exposure. For example, it is knowns that the tongue may serve as a reservoir for pathogens associated with oral diseases in smokers (Alexandridi F, Tsantila S, Pepelassi E. Smoking cessation and response to periodontal treatment. Aust Dent J. 2018 Jun;63(2):140-149*).* In the case of smokers, there is an association between tobacco consumption and bad breath (Ford PJ, Rich AM. Tobacco Use and Oral Health. Addiction. 2021 Dec;116(12):3531-3540*).* This relation is partly due to alterations in the oral microbiota and the production of malodorous compounds. In fact, it has been found that in the oral cavity of smokers there is an increase in bacteria known to contribute to bad breath; these bacteria produce certain molecules, including hydrogen sulfide (H₂S), methyl mercaptan (CH₃SH), and dimethyl sulfide (CH₃SCH₃). At the same time, there is a decrease in beneficial bacteria like Lactobacilli, particularly *Lactobacillus reuteri* (*Biedermann, et. al.*)*.* Furthermore, most *Lactobacillus* species are considered to provide broad-spectrum protection against pathogenic infections through the production of lactic acid and bacteriocins. On the other hand, *Streptococcus mutans, Porphyromonas gingivalis,* and *Fusobacterium nucleatum,* when present in excess, metabolize organic matter generating volatile sulphur compounds (VSC) known for their unpleasant odour. These compounds include hydrogen sulphide (H₂S), methanethiol (CH₃SH), dimethyl sulphide (CH₃SCH₃), methylthiomethane (CH₃SCH₃), methyl mercaptan, and ethyl mercaptan (Tangerman A. Measurement and biological significance of the volatile sulfur compounds hydrogen sulfide, methanethiol and dimethyl sulfide in various biological matrices. J Chromatogr B Analyt Technol Biomed Life Sci. 2009 Oct 15;877(28):3366-77)*.*

It has been demonstrated in vitro that the presence of specific chemicals or metabolites is capable of directly reducing or inhibiting the growth of particular bacterial species. The biosynthesis of biogenic amines (BA) (cadaverine, putrescine, spermine, spermidine, trimethylamine, and tyramine) may enable various pathogenic bacteria to survive and plays a significant role in the destabilization of *Lactobacillus* spp (Sobel, J. D. et al. Diagnosing vaginal infections through measurement of biogenic amines by ion mobility spectrometry. Eur. J. Obstet. Gynecol. Reprod. Biol. 163, 81-84 (2012*)*)*.* An abundance of various biogenic amines (polyamines) among smokers compared to non-smokers is correlated to a decrease in *Lactobacillus reuteri* in the oral tract of smokers. Biogenic amines (BA) are unique molecules carrying one or more amino groups (NH₂). These molecules are essential for the physiology of both mammals and bacteria and are tightly regulated in cellular metabolic processes. Molecules such as cadaverine, putrescine, agmatine, and tryptamine play a role in the metabolism of essential amino acids, including tryptophan and lysine. Their accumulation indicates changes or disruptions in these metabolic systems. In particular, the unpleasant odours emitted by cadaverine and putrescine can be identified as indicators of tissue decomposition associated with death or bacterial contamination (Nelson TM, Borgogna JC, Michalek RD, Roberts DW, Rath JM, Glover ED, Ravel J, Shardell MD, Yeoman CJ, Brotman RM. Cigarette smoking is associated with an altered vaginal tract metabolomic profile. Sci Rep. 2018 Jan 16;8(1):852)*.*

Biogenic amines in the mouth may promote the growth of bacterial species other than *Lactobacillus,* and thus allowing the colonization of a more diversified bacterial community. First, the decarboxylation reactions of amino acids producing biogenic amines result in the consumption of intracellular hydrogen ions and are a well-described mechanism of bacterial acid resistance and mitigation. The consumption of hydrogen ions is considered to be the main barrier to pathogen growth and mitigating the bactericidal effect of *Lactobacillus reuteri* (Kanjee, U. & Houry, W. A. Mechanisms of acid resistance in Escherichia coli. 67, 65-81 (2013*);* Nasrallah, G. K. et al. Legionella pneumophila requires polyamines for optimal intracellular growth. 193, 4346-4360 (2011*);* Jelsbak, L., Tomsen, L. E., Wallrodt, I., Jensen, P. R. & Olsen, J. E. Polyamines are required for virulence in Salmonella enterica serovar Typhimurium. PLoS ONE 7, e36149 (2012*);* Goytia, M. & Shafer, W. M. Polyamines can increase resistance of Neisseria gonorrhoeae to mediators of the innate human host defense. Infection and immunity 78, 3187-3195 (2010*)*)*.*

It should be highlighted that not only cigarette smokers show the above-described oral disorders: also, people which chew tobacco, or which use electronic cigarettes, nicotine pouches or other nicotine related products experience disorders that can overlap with those disclosed above in relation to cigarette smokers. This is because nicotine itself can cause inflammation, dysbiosis or other specific oral disorders.

Therefore, the above-mentioned disorders may occur, in general, for any nicotine user, regardless of the specific nicotine product used. What is needed is a solution to treat oral disorders, or oral conditions, that imply oral inflammation and/or dysbiosis, particularly if associated with chronic nicotine use.

### Summary of the invention

Object of the present invention is to provide a topical oral composition for preventing or treating oral disorders, or oral conditions, related to oral inflammation and/or dysbiosis.

Another object of the present invention is to provide a topical oral composition for preventing or treating oral disorders, or oral conditions, associated with chronic nicotine use.

The present invention, in a first aspect thereof, relates to a topical oral composition according to claim 1.

In particular, claim 1 relates to a topical oral composition comprising *Lactobacillus reuteri* and one or more polyphenols.

In the context of the present invention, a topical oral composition refers to any formulation intended for direct, localized application within the oral cavity, including the teeth, gums, tongue, buccal mucosa, and the oropharyngeal region-particularly the pharynx. A topical oral composition is designed to act topically, meaning it exerts the effect at the site of contact without being intended for systemic absorption. A topical oral composition may be formulated in various physical forms, such as pastes, gels, liquids, sprays, films, pouches or dissolvable solids, depending on the target site.

According to the present invention *"one or more polyphenols"* is to be intended as any polyphenol or any mixture of two or more polyphenols.

Said one or more polyphenols may be selected from: flavonoids (for example quercetin, catechins, anthocyanins), phenolic acids (for example caffeic acid, vanillic acid, ferulic acid), polyphenolic amides (for example capsaicinoids and avenanthramides), secoiridoids (for example oleuropein and oleocanthal), phenolic alcohols (hydroxytyrosol, tyrosol) and other polyphenols (as stilbenes, lignans, resveratrol).

In the preferred embodiment said one or more polyphenols are olive tree polyphenols, as oleuropein, hydroxytyrosol, tyrosol.

For example, the topical oral composition comprises an olive tree extract that, in turn, comprises olive tree polyphenols. The olive tree extract may be produced by olive oil, leaves, olives according to methods known in the art.

Preferably, the weight ratio of *Lactobacillus reuteri* to olive tree polyphenols ranges from 1:80 to 1:2. For example, the weight ratio of *Lactobacillus reuteri* to olive tree polyphenols may be a range selected from: 1: 80, 2: 80, 3: 80, 5: 80, 1: 15, 2: 15, 3: 15, 5: 15, 1: 10, 2: 10, 5: 10.

For example, *Lactobacillus reuteri* is 1 mg, or 2 mg, or 3 mg, or 5 mg while olive tree polyphenols are 10 mg, 15 mg, 80 mg.

It is to be understood that the topical oral composition may comprise *Lactobacillus reuteri* and olive tree polyphenols in amounts or weight ratios different from those specifically disclosed herein, without departing from the scope of the present invention.

Preferably *Lactobacillus reuteri* is heat inactivated and/or tindalized. *Lactobacillus reuteri* may exert its beneficial activity even in an inactivated form, acting as a postbiotic.

The topical oral composition according to the present invention may be in solid form (as a powder) o in liquid form.

For example, it is possible to produce a nicotine pouch comprising the topical oral composition according to the present invention, wherein the topical oral composition is in solid form, in particular as a powder. In such a nicotine pouch, *Lactobacillus reuteri* may be present from 0.30% to 1.53% by weight and olive tree polyphenols from 0.30% to 1.53% by weight.

As alternative, it is also possible to produce a liquid formulation comprising the topical oral composition according to the present invention which is suitable for administration by spray, drops, rinsing, or gargling.

In general, the topical oral composition may be used for realizing medicines, dietary supplements or medical device.

The topical oral composition according to the present invention is suitable to be administered to a person:
- as anti-inflammatory agent for oral disorders related to nicotine-containing products, or
- as agent for restoring microbial balance following chronic nicotine exposure, or
- for promoting and/or maintaining oral microbial homeostasis (eubiosis).
For this purpose, the topical oral composition may be:
a) in liquid form and administered by spray, drops, rinsing, or gargling; or
b) in solid form and administered in the form of a pouch.

The topical oral composition may be administered once per day, twice per day or up to three times per day. Preferably, the topical oral composition is administered after the use of a nicotine-containing product or during the use of a nicotine-containing product (as happens with the use of nicotine pouches).

According to the present invention, *Lactobacillus reuteri* and said one or more polyphenols are administered separately or co-administered in a single formulation.

According to the present invention the term *"nicotine-containing products"* refer to any product comprising nicotine, regardless of whether it also includes tobacco. Non-limiting examples of nicotine-containing products include cigarettes, cigars, cigarillos, chewing tobacco, snuff, snus, nicotine pouches, gums, lozenges, sprays, and inhalers, as well as electronic nicotine delivery systems (ENDS) including e-cigarettes, e-liquids, and heated tobacco products.

As used herein, the term *"nicotine user"* refers to an individual who consumes, or uses, a nicotine-containing product, for example one or more of the aforementioned nicotine-containing products. Correspondingly, the term *"non-nicotine user"* refers to an individual who does not consume, or use, nicotine-containing products.

It is also disclosed that the topical oral composition according to the present invention is for use as medicament for oral disorders related to nicotine-containing products, i.e. oral disorders which are caused by or associated with nicotine-containing products. These oral disorders may be identified also as nicotine-associated oral pathologies or nicotine-related oral mucosal disorders.

It is further disclosed that the topical oral composition according to the present invention is for use as medicament in a method of treatment of one or more of the following pathological oral disorders: gingivitis, early or moderate stage periodontitis, oral candidiasis, chronic inflammation of the oral and/or oropharyngeal mucosa, inflammatory stomatitis, xerostomia, oral microbiota dysbiosis, chronic halitosis of microbial origin, mild Streptococcus pyogenes-induced pharyngitis or pharyngostomatitis.

These oral disorders may be induced by, or may be related to, the use of nicotine-containing products or may also be independent form the use of nicotine; for example, the topical oral composition according to the present invention may be used as medicament for mild *Streptococcus pyogenes-*induced pharyngitis or pharyngostomatitis in a person that does not use nicotine-containing products.

Preferably, the topical oral composition comprises from 1 mg to 3 mg of *Lactobacillus reuteri* and from 10 mg to 80 mg of olive tree polyphenols.

Preferably, said topical oral composition is:
a) in liquid form and is administered by spray, drops, rinsing, or gargling; or
(b) in solid form and is administered in the form of a pouch.

Preferably said topical oral composition is administered once per day, twice per day or up to three times per day.

When used as medicament *Lactobacillus reuteri* and said one or more polyphenols are administered separately or co-administered in a single formulation.

In a further aspect of the present invention, the topical oral composition may be used in the treatment of one or more of the following non-pathological oral conditions: occasional (non-clinical) halitosis, oral irritation due to contact with nicotine-containing products, alteration of the oral microbial flora due to active or passive smoking; post-smoking-related oral dryness, prevention of dysbiosis and inflammation in current or former smokers, support for oral health during nicotine withdrawal, support for oral mucosal homeostasis under conditions of prolonged exposure to irritative agents such as tobacco and nicotine.

### Brief list of the figures

Further characteristics and advantages of the invention will be better highlighted by examining the following detailed description of its preferred, but not exclusive, embodiments depicted by way of non-limiting example, with the support of the appended drawings, wherein:
- Figure 1A shows an electrophoresis agarose gel of bacterial 16s rRNA comparing the results of a PCR analysis of (from left to right) samples taken from a control group, from a tobacco smokers' group before treatment with a topical oral composition according to the present invention and from the same tobacco smokers' group after treatment with a topical oral composition according to the present invention;
- Figure 1B shows an electrophoresis agarose gel of bacterial 16s rRNA comparing the results of a PCR analysis of (from left to right) samples taken from a control group, from nicotine pouch users' group before treatment with a topical oral composition according to the present invention and from the same nicotine pouch users' group after treatment with a topical oral composition according to the present invention;

- Figure 2 shows three Petri dishes with microbial cultures from non-nicotine users;
- Figure 3 shows three Petri dishes with microbial cultures from cigarette users;
- Figure 4 shows three Petri dishes with microbial cultures from nicotine pouch users;
- Figure 5 shows a comparison between two Petri dishes with microbial cultures taken from a cigarette user volunteer before and after the treatment.
- Figure 6 shows a comparison between the front and the rear side of a Petri dish cultured with *Streptococcus pyogenes* isolated from a non-nicotine user and the front and the rear side of a Petri dish cultured with *Streptococcus pyogenes* after 5 days with a treatment with a topical oral composition according to the present invention;
- Figure 7 is a diagram showing normalized inflammation percentage in nicotine users and non-nicotine users;
- figure 8 is a diagram showing normalized inflammation percentage in nicotine users at T1, i.e. before the treatment with a topical oral composition according to the present invention, and at T2, i.e. after the treatment with a topical oral composition according to the present invention

### Detailed description of the invention

It is disclosed an embodiment of the composition according to the present invention comprising olive tree polyphenols and *Lactobacillus reuteri.* The composition is intended for application in the oral cavity and it may be therefore considered a topical oral composition. Optionally, but not necessarily, the composition may be ingested by the user.

In the composition *Lactobacillus reuteri* is at least 1 mg; for example, *Lactobacillus reuteri* is 1 mg, 2 mg, 3 mg, 5 mg.

In the composition olive tree polyphenols are at least 10 mg; for example, olive tree polyphenols are 10 mg, 15 mg, 80 mg but also 1 gram or 2 grams.

The composition may be in liquid form and formulated as a solution or as a tincture that can be applied in the oral cavity as a spray or by drops.

Otherwise, the composition may be in a solid form and formulated into a capsule, a tablet, a chewable tablet, an effervescent tablet or into a powder to be included in a nicotine pouch.

If needed, the composition in liquid or solid form can be dissolved or dispersed in water and then used to perform mouth rinses or gargles.

The composition may be applied in the oral cavity once a day or two or three times a day, preferably after each use of a nicotine product (after smoking or after the use of nicotine pouch).

For example, the composition in powder form can be used as a component of a nicotine pouch. As is known, the nicotine pouch may comprise wax fibres, water, flavouring, humectant (E422), flavour enhancers (coconut oil, salt), nicotine (1.4% by weight) sweeteners (xylitol, E950) and acidity regulator.

It is possible to realize a specific formulation or a specific product (for example dietary supplements, medical devices or medicines) comprising the composition herein disclosed by applying the knowledge available in the art.

In general, *Lactobacillus reuteri* and olive tree polyphenols are food grade. i.e. they are suitable for food use.

Preferably, *Lactobacillus reuteri* is heat inactivated and/or tindalized according to know methods (Cauquil, M.; Olivry, T. Immunomodulating Effects of Heat-Killed Lactobacillus rhamnosus and Lactobacillus reuteri on Peripheral Blood Mononuclear Cells from Healthy Dogs. Vet. Sci. 2025, 12, 226*;* Jansseune, S.C.G.; Lammers, A.; Hendriks, W.H.; van Baal, J.; Wierenga, P.A. The Heat Treatment of a Lactobacilli Probiotic to Derive a Postbiotic Has Minor Effects on the Metabolomic Profile. Fermentation 2025, 11, 87*.*)*.*

*Lactobacillus reuteri* may be dried in powdered form.

In general, also olive tree polyphenols are food grade, i.e. they are suitable for food use.

Olive tree polyphenols may be in freeze dried powdered form.

Preferably olive tree polyphenols comprise hydroxytyrosol, oleuropein and tyrosol.

For example, it may be used an olive tree extract comprising about 40% of olive tree polyphenols. The olive tree extract may be prepared applying the knowledge availabe in the art (Debs, E.; Abi-Khattar, A.-M.; Rajha, H.N.; Abdel-Massih, R.M.; Assaf, J.-C.; Koubaa, M.; Maroun, R.G.; Louka, N. Valorization of Olive Leaves through Polyphenol Recovery Using Innovative Pretreatments and Extraction Techniques: An Updated Review. Separations 2023, 10, 587*;* Abi-Khattar AM, Rajha HN, Abdel-Massih RM, Maroun RG, Louka N, Debs E. Intensification of Polyphenol Extraction from Olive Leaves Using Ired-Irrad®, an Environmentally-Friendly Innovative Technology. Antioxidants (Basel). 2019 Jul 18;8(7):227*;* Alejandra Bermúdez-Oria, Africa Femández-Prior, Maria Luisa Castejón, Guillermo Rodríguez-Gutiérrez, Juan Fernández-Bolaños, "Extraction of polyphenols associated with pectin from olive waste (alperujo) with choline chloride". Food Chemistry, Volume 419, 2023,136073*.;* Athanasiadis V, Voulgaris A, Katsoulis K, Lalas SI, Roussis IG, Gortzi O. Development of Enriched Oil with Polyphenols Extracted from Olive Mill Wastewater. Foods. 2023 Jan 21;12(3):497)*.*

It is also disclosed that a composition comprising olive tree polyphenols and *Lactobacillus reuteri* is intended to be used for preventing or treating oral disorders, or oral conditions. In general, the composition is used to prevent or treat oral inflammation and/or oral dysbiosis in people who use nicotine.

The nicotine use is related to products containing nicotine and may be, for example, tobacco cigarettes, electronic cigarettes or alternative nicotine products as nicotine pouches.

According to the invention, olive tree polyphenols and *Lactobacillus reuteri* may be administered separately (i.e., sequentially) or co-administered in a single formulation (i.e., simultaneously).

The composition can prevent or treat non-pathological or pathological oral disorders.

For example, the composition may be used to prevent or treat one or more of the following pathological oral conditions:
- gingivitis;
- periodontitis (early or moderate stage);
- oral candidiasis;
- chronic inflammation of the oral and/or oropharyngeal mucosa;
- inflammatory stomatitis;
- xerostomia secondary to tobacco use, pharmacological agents, or other etiological factors;
- oral microbiota dysbiosis, either clinically suspected or diagnostically confirmed;
- chronic halitosis of microbial origin;
- mild *Streptococcus* pyogenes-induced pharyngitis or pharyngostomatitis.

The composition may be used also to prevent or treat one or more of the following non-pathological oral conditions:
- occasional (non-clinical) halitosis;
- oral irritation due to contact with nicotine-containing products (e.g., pouches, e-cigarettes);
- alteration of the oral microbial flora due to active or passive smoking;
- post-smoking-related oral dryness;
- prevention of dysbiosis and inflammation in current or former smokers;
- support for oral health during nicotine withdrawal;
- support for oral mucosal homeostasis under conditions of prolonged exposure to irritative agents such as tobacco, alcohol, or chemical compounds.

### Experimental studies

In order to assess the efficacy of the composition two experimental studies were carried out.

During these experimental studies two dietary supplements were administered to the volunteers:
- a first commercial product in liquid form comprising olive tree polyphenols as single active component;
- a second commercial product in solid form comprising *Lactobacillus reuteri* as single active component.
For the purposes of the description of the experimental studies, the combined administration of the two dietary supplements shall be referred to as 'treatment'. The dosage was: 3 mg of *Lactobacillus reuteri* (equivalent to 10 billion of Colony-forming unit CFU) and 80 mg of olive trees polyphenols after the use of nicotine products and maximum three times a day.
Administration mode: rinsing the oral cavity with both solutions for about 10 seconds, followed by ingestion.

### Experiment 1

### Recruitment and Data Collection

Volunteer participants in the study were recruited among adults aged 21 to 29. In particular, nine subjects were selected at the Magi Balkans Centre in Tirana: non-smokers, smokers, and nicotine pouch users. In this group, three participants were traditional cigarette smokers (7-10 cigarettes per day), three participants were nicotine pouch users (5-7 pouches per day), three participants were non-nicotine users, i.e. neither smokers neither nicotine pouch users. The non-nicotine users represented the control group. The participants of the three groups were of the same age range and for each group two participants were males and one was female. Below is the list (table 1):

**Table 1**

| **ID** | **Date of Birth** | **Code** | **Habit** |
|---|---|---|---|
| A.B. | 22.05.1997 | 1C46635C8 | Non-smoker |
| J.N. | 06.07.1998 | 1B74946C8 | Non-smoker |
| S.B. | 26.02.1994 | 1A1D41CC9 | Non-smoker |
| O.S. | 30.11.1994 | 1A698D0C9 | Smoker |
| D.C. | 27.03.1995 | 1B87A73C8 | Smoker |
| S.H. | 22.02.2002 | 1BA443748 | Smoker |
| H.P. | 29.02.2000 | 1BF08EB48 | nicotine pouch users |
| E.Z. | 22.01.1998 | 1C0D2AEC8 | nicotine pouch users |
| L.L. | 17.07.1997 | 1AA2C57C9 | nicotine pouch users |

Whit regard to the evaluation by questionnaire, in the first five days of the experiment participants underwent an initial observational phase prior to intervention. In practice subjects maintained their nicotine-use, or nicotine-free, habit without any changes, i.e. no treatment was given to any of the participants. At the end of these first five days a questionnaire was given to the volunteers in order to assess any possible oral disorder connected to the use of nicotine. In particular, the questionnaire asked to rate from 0 to 10 the grade of the following oral disorders: halitosis, gingival inflammation, oral cavity inflammation, dry mouth.

After the initial observational phase, a ten-day study was performed. **In** an initial five-day period, no treatment was administered to the nicotine users. In a subsequent five-day period, the nicotine users received the treatment. At the end of the ten-day study, the same questionnaire given at the beginning was administered to all the participants in order to assess whether they had experience improvements after the use of the treatment for five days.

### Metabolomics and Proteomics Analysis

Metabolomics and proteomics analysis involved liquid chromatography (LC) with a Phenomenex Jupiter C18 column. Chromatographic separation was carried out using a gradient eluent over a 15-minute period. Mass spectrometry analysis was performed using a high-capacity ion trap mass spectrometer, HCT Ultra, with electrospray ionization. This analysis was conducted in both positive and negative ion modes. The acquired mass spectrometry data were processed and analysed using the SANIST software, allowing for the identification, characterization, and quantification of analytes.

At the metabolomic level, Pipecolic Acid, Tyramine, N-Acetylcadaverine, N-Acetylspermine, Methylthiophene, Spermidine, 5-oxoproline, and Histidine were analysed. In fact, these metabolites are biogenic amines (or can be considered as their precursor) and, therefore, can be useful biomarkers for oral disorders, as periodontitis or oral dysbiosis.

Proteomic analyses examined the following salivary biomarkers: CXCL10, CRP, alpha-amylase, NGAL, transferrin, MIP-1a, MCP-1, SAA, PAS1, MRP8 (S100A8), MRP14 (S100A9), IgA, and Haptoglobin. Also, these proteins can be used to detect oral disorders as oral inflammation.

### Microbiomics Analysis

During the experiment, various microorganisms have been isolated and cultured from lingual and throat swabs, as well as pus samples. These microorganisms included *Staphylococcus aureus* from the pus culture and a mixture of bacteria from throat swabs, which included *Streptococcus, Staphylococcus,* and *Neisseria* species. They were cultivated in standard Brain Heart Infusion (BHI) liquid medium and subjected to a 4-hour incubation at 37°C to reach the logarithmic growth phase. Four sets of tubes were then prepared, with some containing 2 ml of BHI medium and others having 1 ml of BHI medium and 1 ml of a specific formulation. All tubes were inoculated with 100 µl of the logarithmic growth phase cultures, resulting in different microorganism/medium combinations. Following an overnight incubation at 37°C, 1 µl of each culture was plated on solid media in a divided Petri dish, with one side containing BHI medium and the other BHI medium with the formulation. Columbia Blood Agar (Biolife) was used for all other cultures.

Growth differences were subsequently assessed and reported in the results.

From these subjects, several cultures were prepared to assess the growth of pathogenic and non-pathogenic microorganisms. Subjects were provided with a sterile container and 20 ml vials of sterile water, which they used to rinse their oral cavity and then brought to the laboratory. This allowed to evaluate the growth or inhibition of oral cavity microorganisms, both pathogenic and non-pathogenic. The following tests were performed:
1. evaluation of the growth of oropharyngeal microorganisms in three control subjects;
2. evaluation of the growth of oropharyngeal microorganisms in three tobacco smokers;
3. evaluation of the growth of oropharyngeal microorganisms in nicotine pouch users;
4. evaluation of the growth of oropharyngeal microorganisms in three tobacco smokers and nicotine pouch users using the treatment.

The protocol was the following: 1 ml was taken from the sterile container containing 20 ml of oral rinse from the nine subjects and inoculated into 4 ml of Brain Heart Infusion (BHI) liquid medium, which is a universal medium. The composition of the Brain Heart Infusion (BHI) medium is: enzymatic digest of animal tissues 10.0 g/l, dehydrated calf brain infusion 12.5 g/l, dehydrated beef heart infusion 5.0, glucose 2.0 g/l, sodium Chloride 5.0 g/l, anhydrous disodium hydrogen phosphate 2.5 g/l. Final pH 7.4 ± 0.2 at 25°C.

All the nine subjects collected two samples: the first as described above, and the second after the nicotine users had performed daily rinses for two weeks with the treatment. The fifteen cultures with microorganisms were then incubated at 37°C for 4 hours to reach the optimal growth phase. To assess their growth, 1 µl of each individual culture was seeded on solid Columbia Blood Agar (Biolife) and incubated for 48 hours at 37°C. Columbia Agar Base composition: Peptone complex 10 gr/L, Tryptose 10 gr/L, Peptone 3 gr/L, Maize starch 1 gr/L, Sodium chloride 5 gr/L, Agar 12 gr/L.

### Amplification of Bacterial DNA

Saliva was collected, and DNA was extracted from the saliva before and after the study to assess whether the bacterial load changes after the use of the treatment. Saliva extraction and DNA isolation and amplification were performed using standard protocols. Universal primers were used for the amplification of the extracted bacterial DNA; this was done to evaluate possible differences in microbial growth with or without the use of the treatment.

### Results

The metabolomics and proteomics results showed an increase in inflammatory markers in the nicotine users' group.

**Table 2**

| **Metabolites** | **Ratio Smokers vs. Controls** | **Ratio Nicotine Pouches Users vs. Controls** |
|---|---|---|
| Pipecolic Acid | 1.2 | 1.1 |
| Tyramine | 1.5 | 1.4 |
| N-Acetylcadeverine | 1.7 | 1.2 |
| N-AcetylSpermine | 1.3 | 1.2 |
| Metiltiophene | 1.4 | 1.3 |
| Spermidine | 1.9 | 1.6 |
| 5-oxoproline | 1.2 | 1.3 |
| Histidine | 1.3 | 1.2 |

Table 2 above shows a comparison of nicotine users vs. control group. These ratios indicate how the levels of these metabolites compare in smokers and nicotine pouches users relative to the control group. In general, the ratios are higher in both the smokers and nicotine pouches users' groups, suggesting an alteration in the metabolite profiles which may be indicative of the impact of smoking and nicotine on oral inflammation and dysbiosis.

In particular:
- both smokers and nicotine pouches users have a higher ratio of Tyramine (1.5 and 1.4, respectively), indicating increased levels of Tyramine relative to the control group;
- smokers have a higher ratio of N-Acetylcadaverine (1.7) compared to nicotine pouches users (1.2), but both groups show an increase in levels compared to the control group;
- both groups exhibit a similar increase in N-Acetylspermine levels, with ratios of 1.3 in smokers and 1.2 in nicotine pouches users;
- the ratio of Metiltiophene is higher in both groups (1.4 for smokers and 1.3 for nicotine pouch users), indicating elevated levels compared to the control group.
- spermidine levels are notably higher in both smokers (1.9) and nicotine pouches users (1.6) when compared to the control group.

Table 3 below shows a comparison of inflammatory and immune proteomic markers ratios in smokers and nicotine pouches users vs. control group. These ratios indicate how the levels of these markers compare in smokers and nicotine pouches users relative to the control group. Generally, the ratios are higher in both groups, suggesting alterations in the levels of these biomarkers in response to smoking and nicotine pouch consumption. The specific changes vary for each marker, but, in general, they reflect differences in inflammatory and immune responses.

**Table 3**

| **Markers** | **Ratio Smokers vs. Controls** | **Ratio Nicotine Pouches Users vs. Controls** |
|---|---|---|
| CXCL10 | 1.5 | 1.3 |
| CRP | 1.4 | 1.2 |
| Alfa-amylase | 1.3 | 1.1 |
| NGAL | 1.6 | 1.4 |
| Transferrin | 1.2 | 1.3 |
| MIP-1a | 1.4 | 1.2 |
| MCP-1 | 1.5 | 1.3 |
| SAA | 1.2 | 1.4 |
| PAS1 | 1.3 | 1.1 |
| MRP8 (S100A8) | 1.5 | 1.3 |
| MRP14 (S100A9) | 1.6 | 1.4 |
| IgA | 0.9 | 0.9 |
| Haptoglobin | 1.0 | 1.0 |

### Microbiomics Analysis

Microbiomics analyses were conducted using universal bacterial 16S rRNA primers, along with functional metabolomic analysis. Furthermore, an increase in oral microbial flora was observed in nicotine users. Table 4 below shows a comparison of oral bacterial abundance in nicotine users vs. a control group. These ratios show how the abundance of these specific bacterial species differs in nicotine users compared to the control group. In general, the control group has a significantly higher presence of *Lactobacillus* spp. and *Lactobacillus reuterii,* while the levels of *Streptococcus mutans, Porphyromonas gingivalis,* and *Fusobacterium nucleatum* are notably reduced in nicotine users.

**Table 4**

| **Bacteria** | **Ratio Controls vs. Nicotine users** |
|---|---|
| *Lactobacillus spp.* | 5 |
| *Lactobacillus reuterii* | 7.2 |
| *Streptococcus mutans* | 0.1 |
| *Porphyromonas gingivalis* | 0.3 |
| *Fusobacterium nucleatum* | 0.3 |

### Bacterial Oral Microbiota

The DNA extracted from the saliva of the nine volunteers was amplified using universal primer specific for microbial growth.

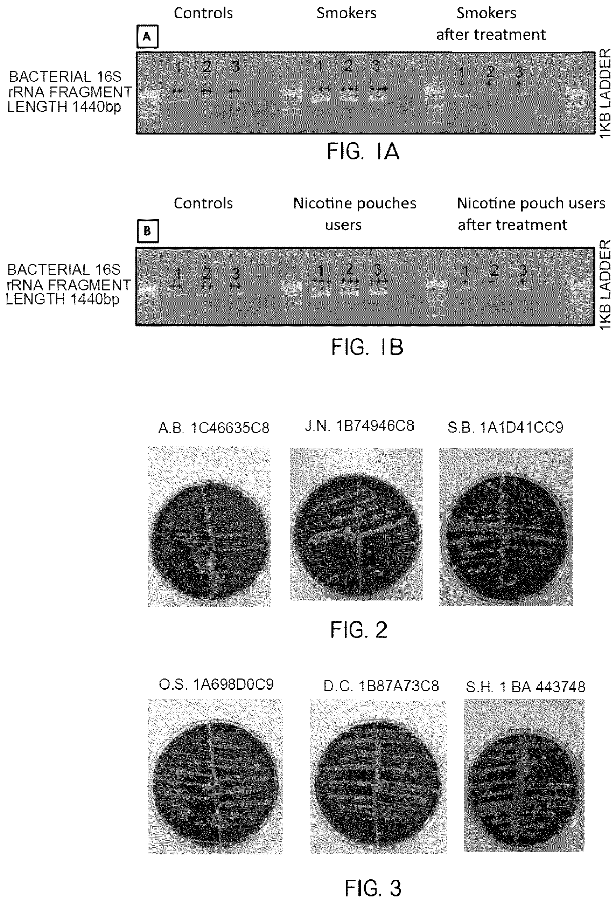

Figures 1A and 1B show the reduction of DNA amplification by PCR of bacterial DNA after using the treatment (the three rightmost wells in A and B panels) in individuals who have smoked tobacco or chewed nicotine pouches. In particular in figure 1A it is shown the difference of bacterial load in tobacco smokers before and after the use of the treatment, versus the control; in figure 1B it is shown the difference of bacterial load in nicotine pouch users before and after the use of the treatment, versus the control.

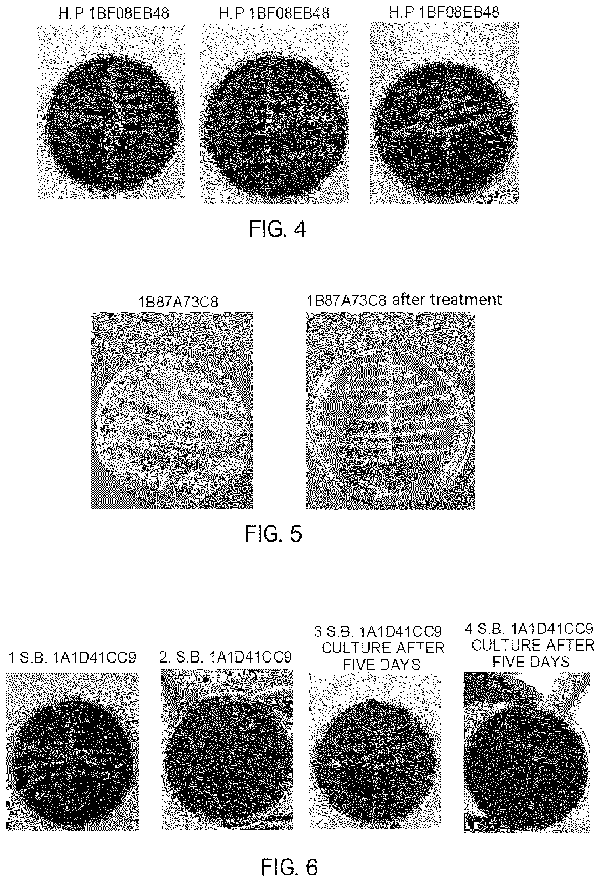

Figure 2 shows bacterial growth in non-nicotine users, figure 3 shows bacterial growth in tobacco smoking patients, figure 4 shows bacterial growth in nicotine pouch users.

In addition, it was possible to observe in a smoking patient (D.C. 1B87A73C8) the growth of *Candida albicans* (oral candidiasis has been extensively described in scientific literature in smoking patients). Thus, 100 µl of the two samples (before and after using the solution) was seeded on Agar Sabouraud Dextrose myceto selective medium (Biolife) and left in thermostat at 37°C for five days. Agar Sabouraud Dextrose (Biolife) composition: Peptone 5 gr./L, Tryptose 5 gr/L, Glucose 40 gr./L, Chloranphenicol gr. 0.015 and Agar 15 gr./L. The different growth between the first sample and the second sample, where the patient rinsed daily (for two weeks) with the treatment could thus be assessed. Decrease of the microorganism load is evident in figure 5.

In the nonsmoking subject S.B. 1A1D41CC9 *Streptococcus pyogenes* was isolated. With regard to figure 6 it was possible to observe after five days of treatment not only the reduction of bacterial load but in particular the reduction of β-haemolytic colonies typical of *Streptococcus pyogenes.*

### Questionnaire results

Table 5 below shows the questionnaire results. The results indicate a reduction of the oral disorders caused by nicotine after the use of the treatment.

**Table 5**

| **Disturbance** | **Value** | **Reduction (%)** |
|---|---|---|
| Halitosis | Before: 6/9 | 67% |
| | After: 2/9 | |
| Gingival inflammation | Before: 5/9 | 60% |
| | After: 2/9 | |
| Oral cavity inflammation | Before: 6/9 | 83% |
| | After: 1/9 | |
| Dry mouth | Before: 7/9 | 57% |
| | After: 3/9 | |

In practice, at the end of the study (after 5 days of treatment), it was found a strong tendency towards a reduction of halitosis (-67%), gingival inflammation (-60%), mouth inflammation (-83%), and dry mouth (-57%).

The research conducted clearly highlights how smoking and oral nicotine use substantially affect oral health, giving rise to inflammatory processes and microbiome imbalances. However, it could be observed that the application of the treatment, which combines olive tree polyphenols and *Lactobacillus reuteri,* was surprisingly effective in restoring microbial balance and normalizing the level disorders. This finding represents a significant advance in oral health promotion, offering a potential improvement in quality of life for nicotine users, with a reduction in oral discomforts such as halitosis, gingival inflammation, and dry mouth.

### Experiment 2

### Recruitment and Data Collection

Volunteer participants (n=108) were recruited in collaboration with the University of SS. Cyril and Methodius in Trnava. The study involved adult volunteers aged 18 to 26 who were divided into two groups: nicotine users (n=54) and non-nicotine users (n=54). In the nicotine users' group, participants used cigarettes, e-cigarettes, nicotine pouches, or a combination of these products. Data on tobacco use frequency, oral hygiene practices, and the prevalence of oral health conditions such as tooth decay, gingivitis, and laryngitis were collected. Each participant completed a questionnaire to gather these data, and a dry buccal swab was collected for metabolomic and proteomic analysis. Additionally, a subgroup of smokers agreed to use the treatment (olive tree polyphenols and *Lactobacillus reuteri*) for 7 days. For this subgroup, data and biological sample were collected at two time points, before (T1) and after the treatment (T2), to assess the efficacy of the treatment. The subjects were divided into two groups: non-nicotine users and nicotine users, as shown in Table 6.

**Table 6.**

| **Characteristics** | **Values** |
|---|---|
| Total number of volunteer subjects | 108 |
| Female | 70 |
| Male | 38 |
| Age range (years) | 18-26 |
| Nicotine users | 54 |
| Non-nicotine users | 54 |

Within the nicotine users' group different types of subgroups were identified (see table 7): 22 participants use only cigarettes, 11 use only e-cigarettes, 11 use only nicotine pouches, and 10 use a combination of these products. The frequency of product usage varies, with 20 participants usage less than 5 times/day, 20 using nicotine products 5 times/day, 7 using nicotine products 10 times/day, and 7 using nicotine products more than 10 times/day. Oral hygiene rituals differ between groups. In the nicotine users' group 31 participants report low oral hygiene, compared to 27 in the non-nicotine users' group. Normal oral hygiene is reported by 22 nicotine users and 26 non-nicotine users, while high oral hygiene is uncommon in both groups (one participant each). Tooth decay, gingivitis, and laryngitis occur in both groups with similar frequencies. Inflammation of the oral cavity is slightly more common in smokers (three cases) compared to non-smokers (one case). The usage of mouthwash is nearly equal between the groups, with 25 nicotine users and 23 non-nicotine users using it regularly.

**Table 7. Data comparison between nicotine users and non-nicotine users**

| **Characteristics** | **Values** | |
|---|---|---|
| **Type of Product** | **Nicotine users' Group (N=54)** | **Non-nicotine users' Group (N=54)** |
| Cigarettes only | **22** | - |
| E-cigarettes only | 11 | - |
| Nicotine Pouches only | 11 | - |
| Mixed | 10 | |

| **Frequency** | | |
|---|---|---|
| Less than 5/day | 20 | - |
| 5 times/day | 20 | - |
| 10 times/day | 7 | - |
| More than 10 times/day | 7 | - |

| **Oral Hygiene Ritual** | | |
|---|---|---|
| Low | 31 | 27 |
| Normal | 22 | 26 |
| High | 1 | 1 |
| **Oral Health Conditions** | | |
| Tooth Decay | 32 | 35 |
| Gingivitis | 6 | 11 |
| Inflammation of Oral Cavity | 3 | 1 |
| Dry Mouth | 2 | 5 |
| Laryngitis | 30 | 28 |
| Bronchitis | 18 | 19 |
| Digestion Problems | 14 | 19 |
| Usage of Mouthwash | 25 | 23 |

Five volunteer smokers agreed to use an oral treatment for seven days, to identify molecular, metabolomic, and proteomic differences between data collected at T1 (before the treatment) and T2 (after seven days of treatment). A comparison of data collected from a subgroup of five smoker volunteers at T1 and T2 reveals the following trends (Table 8). The type of cigarettes used by the participants remained consistent, with four subjects continuing to use only cigarettes and one subject using only nicotine pouches at both time points. The frequency of smoking showed a slight shift, with the number of subjects smoking less than five times per day decreasing from three to two, while those smoking 5 times per day increased from two to three. In terms of oral hygiene rituals, there was a decline in reported hygiene, with four subjects indicating low hygiene at T1, increasing to five at T2. Oral health conditions such as tooth decay, laryngitis, and bronchitis remained unchanged between T1 and T2. However, there was a reduction in the number of subjects reporting digestion problems, decreasing from two at T1 to one at T2. The usage of mouthwash remained consistent, with three participants using it at both time points.

**Table 8. Data comparison among a subgroup of nicotine users at T1 and T2**

| **Characteristics** | **Values** | |
|---|---|---|
| Type of Cigarettes | T1 (N=5) | T2 (N=5) |
| Cigarettes only | 4 | 4 |
| E-cigarettes only | - | - |
| Nicotine Pouches only | 1 | 1 |
| Mixed | - | - |
| Frequency | | |
| Less than 5/day | 3 | 2 |
| 5 times/day | 2 | 3 |
| 10 times/day | - | - |
| More than 10 times/day | - | - |

| **Oral Hygiene Ritual** | | |
|---|---|---|
| Low | 4 | 5 |
| Normal | 1 | - |
| High | - | - |

| **Oral Health Conditions** | | |
|---|---|---|
| Tooth Decay | 2 | 2 |
| Gingivitis | - | - |
| Inflammation of Oral Cavity | 1 | 1 |
| Dry Mouth | - | - |
| Laryngitis | 4 | 4 |
| Bronchitis | 3 | 3 |
| Digestion Problems | 2 | 1 |
| Usage of Mouthwash | 3 | 3 |

### Metabolomics and Proteomics Analysis

Sample extraction and preparation involved collecting dry buccal salivary swabs from each participant. The swabs were used for both metabolomic and proteomic analyses. For metabolomics, liquid chromatography (LC) was performed using a Phenomenex Jupiter C18 column, with chromatographic separation carried out using a gradient eluent over a 15-minute period. Mass spectrometry (MS) analysis was conducted using a high-capacity ion trap mass spectrometer, HCT Ultra, with electrospray ionization in both positive and negative ion modes. The acquired mass spectrometry data were processed and analysed using SANIST software to identify, characterize, and quantify analytes. For proteomic analysis, a shotgun proteomics approach was employed. Proteins extracted from the dry buccal salivary swabs were digested, and the resulting peptides were subjected to LC-MS/MS analysis. The mass spectrometry data were processed to identify and quantify the metabolites and proteins, to identify the profile of the samples. The initial analysis focused on identifying molecular differences between the Smoker Group (N=54) and the Non-Smoker Group (N=54) through metabolomic and proteomic data. Significant variations were observed in several markers, as shown in Table 9.

**Table 9. MS Data comparison between Smoker Group and Non-Smoker Group**

| **Markers** | **Ratio nicotine vs. non-nicotine users** | **P-value** |
|---|---|---|
| **Metabolomics Data** | | |
| Pipecolic Acid | 2,62 | 3,03E-48 |
| Tyramine | 2,43 | 1,42E-44 |
| N-Acetylcadeverine | 14,12 | 7,18E-53 |
| N-AcetylSpermine | 3,64 | 2,64E-49 |
| Metiltiophene | 2,24 | 2,29E-45 |
| Spermidine | 2,98 | 7,59E-49 |
| 5-oxoproline | 1,74 | 2,05E-40 |
| Histidine | 1,36 | 2,56E-27 |

| **Proteomics Data** | | |
|---|---|---|
| CXCL10 | 2,92 | 1,84E-54 |
| CRP | 3,48 | 1,61E-46 |
| Alfa-amylase | 2,22 | 1,77E-40 |
| NGAL | 5,97 | 5,13E-52 |
| Transferrin | 3,91 | 6,48E-48 |
| MIP-1a | 1,96 | 1,73E-42 |
| MCP-1 | 7,18 | 2,89E-50 |
| SAA | 1,67 | 5,55E-38 |
| PAS1 | 2,54 | 7,37E-47 |
| MRP8 (S100A8) | 1,26 | 2,00E-18 |
| MRP14 (S100A9) | 9,96 | 1,68E-47 |
| IgA | 1,14 | 8,27E-08 |
| Haptoglobin | 2,91 | 9,00E-44 |

In the metabolomics analysis, smokers exhibited notably higher levels of several compounds compared to non-smokers, including N-Acetylcadaverine, which showed the largest increase (14.12-fold, P=7.18E-53), followed by N-Acetylspermine (3.64-fold, P=2.64E-49), and Pipecolic Acid (2.62-fold, P=3.03E-48). Other metabolites such as Spermidine, Tyramine, and 5-oxoproline also demonstrated significant increases, with ratios of 2.98, 2.43, and 1.74 respectively, all with highly significant p-values. In the proteomics analysis, the most pronounced differences were observed in MRP14 (S100A9), with nearly a 10-fold increase (P=1.68E-47) in smokers compared to non-smokers. Other proteins, such as MCP-1 (7.18-fold, P=2.89E-50), NGAL (5.97-fold, P=5.13E-52), and CRP (3.48-fold, P=1.61E-46), also showed substantial elevations in the smoker group. These findings indicate a significant alteration in both metabolomic and proteomic profiles in smokers, which could be linked to their smoking status, and which may be indicative of the impact of smoking and nicotine pouch use on oral inflammation and dysbiosis.
The second analysis focused on identifying molecular differences in a subgroup of nicotine users (N=5) at two time points, T1 (before treatment) and T2 (after treatment). The results, presented in Table 10, show significant changes in several markers. In the metabolomic data, notable decreases were observed in N-Acetylcadaverine (1.77-fold, P=0.00043), Pipecolic Acid (1.67-fold, P=1.23E-06), and Spermidine (1.66-fold, P=0.00009) after supplementation, indicating a reduction in the elevated levels of these metabolites. Other metabolites, including N-Acetylspermine, 5-oxoproline, and Histidine, also showed significant reductions, suggesting a reversal of oral inflammation due to the supplementation. Proteomic data revealed substantial changes in several proteins. CXCL10 decreased by 1.84-fold (P=0.00011), while MRP8 (S100A8) and CRP showed reductions of 1.68-fold (P=0.000004) and 1.64-fold (P=0.00056), respectively, indicating an attenuation of inflammation. Other proteins such as NGAL, MCP-1, and Haptoglobin also exhibited significant reductions, suggesting that the supplementation helped in modulating the inflammatory response and potentially improving oral health. These findings highlight the positive molecular impact of the supplementation on both metabolomic and proteomic levels in the smoker subgroup.

**Table 10. MS Data comparison among a subgroup of nicotine users at T1 and T2**

| **Markers** | **Ratio T1/T2** | **P-value** |
|---|---|---|
| **Metabolomics Data** | | |
| Pipecolic Acid | 1,67 | 1,23E-06 |
| Tyramine | 1,51 | 0,00029 |
| N-Acetylcadeverine | 1,77 | 0,00043 |
| N-AcetylSpermine | 1,63 | 0,00002 |
| Metiltiophene | 1,58 | 0,00046 |
| Spermidine | 1,66 | 0,00009 |
| 5-oxoproline | 1,59 | 0,00057 |
| Histidine | 1,55 | 0,001 |

| **Proteomics Data** | | |
|---|---|---|
| CXCL10 | 1,84 | 0,00011 |
| CRP | 1,64 | 0,00056 |
| Alfa-amylase | 1,69 | 0,00095 |
| NGAL | 1,65 | 0,00003 |
| Transferrin | 1,58 | 0,00054 |
| MIP-1a | 1,58 | 0,00027 |
| MCP-1 | 1,63 | 0,00075 |
| SAA | 1,66 | 0,00003 |
| PAS1 | 1,67 | 0,00034 |
| MRP8 (S100A8) | 1,68 | 0,000004 |
| MRP14 (S100A9) | 1,65 | 0,001157 |
| IgA | 1,66 | 0,002154 |
| Haptoglobin | 1,72 | 0,000581 |

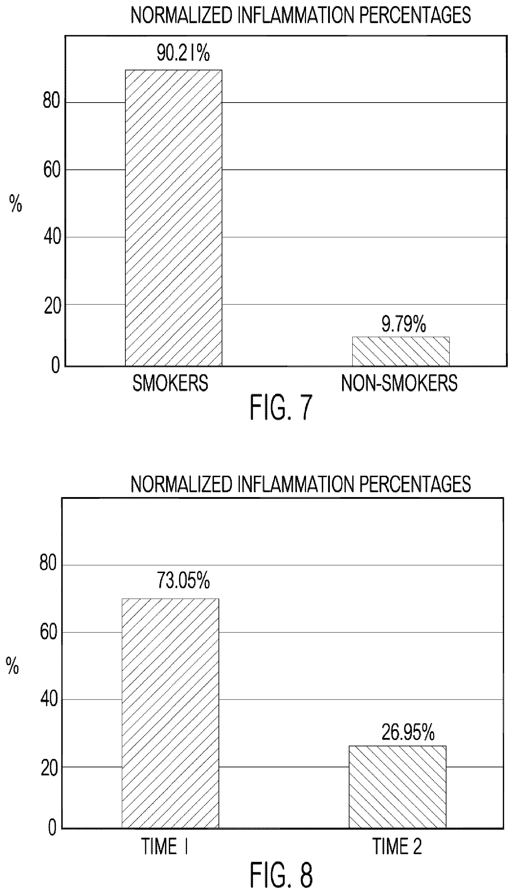

The normalized inflammation percentage in nicotine users and non-nicotine users was calculated and shown in figure 7. The results show a clear predominance of inflammation in nicotine users, with a percentage of 90.21%, compared to only 9.79% in non-nicotine users. This suggests that inflammatory activity is significantly higher in nicotine users, confirming the association between nicotine use and increased inflammatory markers. Also, the inflammation percentages in a group of smoker volunteers at two distinct time points, T1 and T2 has been calculate and reported in Figure 8. The results indicate higher inflammation at T1, with a normalized percentage of 73.05%, compared to 26.95% observed at T2. This may suggest that, over time or in response to the treatment, there has been a reduction in inflammation, as evidenced by the decrease in percentage from T1 to T2.

### Discussion

The data presented provide evidence of the molecular impact of nicotine use on oral health and how targeted treatments can modulate these effects. Our initial analysis comparing nicotine and non-nicotine users revealed significant alterations in both metabolomic and proteomic profiles, with nicotine users exhibiting elevated levels of various metabolites and proteins associated with inflammation and oxidative stress. The metabolomic analysis (Table 9) of the nicotine users' group reveals significant alterations in various metabolites that are closely associated with inflammatory responses and oral dysbiosis. Among the key metabolites identified are N-Acetylcadaverine, N-Acetylspermine, Pipecolic Acid, Tyramine, Methylthiophene, Spermidine, 5-oxoproline, and Histidine. The elevated levels of these compounds in nicotine users compared to non-nicotine users suggest a profound metabolic shift induced by nicotine use contributing to an inflammatory state in the oral environment *(*Satyanarayana M, Rajeswari KR. Biogenic amines in human gingiva in healthy and inflamed states. Indian J Dent Res. 1990 Apr-Sep;2(2-3):170-3).

One of the findings is the significant increase in N-Acetylcadaverine (14.12-fold), N-Acetylspermine (3.64-fold), and Pipecolic Acid (2.62-fold) in the smoker group. These metabolites are linked to polyamine metabolism, which plays a critical role in cell growth, proliferation, and apoptosis. Polyamines like Spermidine and Spermine, as well as their precursors, are known to accumulate in conditions of oral dysbiosis and inflammation. Specifically, the elevated levels of N-Acetylcadaverine, a product of lysine degradation, indicate an imbalance in microbial activity within the oral cavity, potentially leading to halitosis and other oral health issues common in smokers. This dysbiotic environment is likely exacerbated by the reduced presence of beneficial bacteria, such as *Lactobacillus reuteri,* which normally help regulate polyamine levels and maintain oral health *(*Cheng J, Chen P, Song A, Wang D, Wang Q. Expanding lysine industry: industrial biomanufacturing of lysine and its derivatives. J Ind Microbiol Biotechnol. 2018 Aug;45(8):719-734*.).* The increase in Pipecolic Acid is another noteworthy observation. Pipecolic Acid is a lysine metabolite involved in the catabolic pathway of this amino acid. It has been associated with the pathogenesis of periodontitis, a chronic inflammatory condition of the gums. In nicotine users, elevated levels of pipecolic acid may indicate increased lysine degradation due to the proliferation of pathogenic bacteria that favour inflammatory conditions. Its accumulation in the oral cavity of nicotine users suggests enhanced lysine degradation, possibly due to the overgrowth of certain pathogenic bacteria that thrive in the altered oral microbiome of smokers. The elevated levels of this marker in nicotine users align with the findings of Satyanarayana et al., who reported similar increases in patients with periodontitis (Satyanarayana M, and Rajeswari KR). This suggests that smoking may promote conditions favourable to the production of Pipecolic Acid, thereby contributing to the inflammatory processes (Satyanarayana M and Rajeswari KR).

Tyramine, another metabolite that showed a significant increase in nicotine users, is a biogenic amine derived from the amino acid tyrosine. Tyramine and other biogenic amines, such as cadaverine and histamine, are associated with states of microbial dysbiosis. The accumulation of Tyramine in nicotine users may reflect a shift in the composition of the oral microbiota toward bacterial species that produce biogenic amines, and which are overrepresented. This imbalance may contribute to the inflammatory state observed in the oral cavities of smokers, leading to conditions like gingivitis and increased susceptibility to infections (Satyanarayana M and Rajeswari KR). Spermidine, another polyamine, along with 5-oxoproline and Histidine, also showed elevated levels in the nicotine users' group. Spermidine is involved in cellular processes such as DNA stabilization, enzyme regulation, and oxidative stress response. Its increased presence in nicotine users suggests heightened cellular activity, possibly as a response to the oxidative stress induced by smoking. 5-oxoproline, a metabolite linked to the glutathione cycle, and Histidine, an amino acid involved in protein synthesis and immune responses, further indicate a pro-inflammatory state in the oral environment of nicotine users. The elevated levels of these metabolites, as reported in patients with periodontitis, underscore the potential of these molecules as biomarkers for oral inflammation and dysbiosis (Kuboniwa M, Sakanaka A, Hashino E, Bamba T, Fukusaki E, Amano A. Prediction of Periodontal Inflammation via Metabolic Profiling of Saliva. J Dent Res. 2016 Nov;95(12):1381-1386*).*

Importantly, the subgroup analysis of nicotine users who received oral treatment revealed a significant reduction in these pro-inflammatory metabolites. After 7 days of supplementation with the treatment, levels of N-Acetylcadaverine (1.77-fold reduction), Pipecolic Acid (1.67-fold reduction), and Spermidine (1.66-fold reduction) all decreased, indicating an attenuation of the inflammatory state. This indicates that the treatment helped restore metabolic balance in the oral environment, likely by enhancing the protective effects of beneficial bacteria and reducing the dominance of pathogenic species (Cardona F, Andrés-Lacueva C, Tulipani S, Tinahones FJ, Queipo-Ortuño MI. Benefits of polyphenols on gut microbiota and implications in human health. J Nutr Biochem. 2013 Aug;24(8):1415-22 -14*).*

The observed metabolic changes in nicotine users are likely a result of both direct effects of smoking and/or nicotine on the oral microbiome and the indirect effects of smoking/nicotine-induced inflammation. The absence or reduced activity of beneficial bacteria, such as *Lactobacillus reuteri,* which has anti-inflammatory properties, may allow for the proliferation of pathogenic bacteria that drive the production of pro-inflammatory metabolites. This imbalance not only disrupts the oral microbiome but also contributes to the chronic inflammatory state observed in nicotine users, increasing their risk of developing severe periodontal diseases (Han YW, Wang X. Mobile microbiome: oral bacteria in extra-oral infections and inflammation. J Dent Res. 2013 Jun;92(6):485-91*;* Satyanarayana M, and Rajeswari KR). Similarly, the proteomic data demonstrated substantial increases in inflammatory markers such as MRP14, MCP-1, NGAL, and CRP. In this context, biomarkers such as CXCL10, CRP, NGAL, MIP-1α, MCP-1, SAA, PAS1, MRP8, and MRP14 represent indicators of oral inflammation.

From Table 9, it is possible to observe that the nicotine users' group shows higher ratios for most markers, indicating elevated levels when compared to the control non-nicotine users' group. The observed ratios suggest that the consumption of products such as cigarettes, e-cigarettes and nicotine pouches have an impact on inflammatory and immune responses, leading to differences in the levels of these biomarkers. CXCL10 showed a significant decrease following supplementation (1.84-fold reduction, P=0.00011). CXCL10 is a chemokine known to be upregulated in response to the presence of pathogens such as *Fusobacterium nucleatum,* a bacterium associated with periodontitis. Elevated levels of CXCL10 have been linked to inflamed periodontal tissues, suggesting that the high levels of this protein in smokers may indicate the presence of such pathogens and the resultant inflammation. The observed reduction in CXCL10 post-treatment (Table 10) suggests that the treatment may help mitigate the inflammatory response potentially driven by these pathogenic bacteria (Rath-Deschner B, Memmert S, Damanaki A, de Molon RS, Nokhbehsaim M, Eick S, Kirschneck C, Cirelli JA, Deschner J, Jäger A, Nogueira AVB. CXCL5, CXCL8, and CXCL10 regulation by bacteria and mechanical forces in periodontium. Ann Anat. 2021 Mar; 234:151648*).* Similarly, C-reactive protein (CRP), a well-known marker of systemic inflammation, also showed a significant reduction (1.64-fold, P=0.00056) after the treatment. CRP levels have been positively correlated with the severity of gingival inflammation, and its decrease is often associated with improved oral health and reduced systemic inflammation. Studies have shown that intensive oral hygiene can lead to a significant reduction in CRP levels, reinforcing its role as a biomarker of oral inflammation. The decline in CRP observed (Table 10) in our study supports the hypothesis that reducing oral inflammation through targeted treatment can have broader anti-inflammatory effects (Perić M, Marhl U, Gennai S, Marruganti C, Graziani F. Treatment of gingivitis is associated with reduction of systemic inflammation and improvement of oral health-related quality of life: A randomized clinical trial. J Clin Periodontol. 2022 Sep;49(9):899-910*).*

Another important protein, NGAL (Neutrophil Gelatinase-Associated Lipocalin), exhibited a significant reduction (1.65-fold, P=0.00003) after supplementation. NGAL is a biomarker that has been associated with inflammation, particularly in severe periodontal diseases. Elevated levels of NGAL in saliva have been reported in individuals with periodontitis compared to healthy controls. The reduction in NGAL levels (Table 10) following treatment suggests an attenuation of inflammatory processes in the oral cavity, likely contributing to the overall improvement in oral health observed in the study participants (Morelli T, Stella M, Barros SP, Marchesan JT, Moss KL, Kim SJ, Yu N, Aspiras MB, Ward M, Offenbacher S. Salivary biomarkers in a biofilm overgrowth model. J Periodontol. 2014 Dec;85(12):1770-8*).* MCP-1 and MIP-1α, both chemokines involved in monocyte and macrophage recruitment, also showed significant decreases (1.63-fold, P=0.00075 and 1.58-fold, P=0.00027, respectively). These proteins have been linked to dysbiosis in the oral microbiome and are associated with the development of chronic periodontitis. Elevated levels of MCP-1 and MIP-1α correlate with increased bacterial load and severity of periodontal disease. The reductions in these markers (Table 10) post-treatment suggest that the intervention may help restore a more balanced microbial environment and reduce the inflammatory response characteristic of periodontal diseases (Nisha KJ, Suresh A, Anilkumar A, Padmanabhan S. MIP-1α and MCP-1 as salivary biomarkers in periodontal disease. Saudi Dent J. 2018 Oct;30(4):292-298*).* Calprotectin, a complex of MRP8 and MRP14, is another critical inflammatory marker that decreased significantly (1.68-fold reduction for MRP8, P=0.000004; 1.65-fold reduction for MRP14, P=0.001157) after supplementation. Calprotectin is highly expressed during inflammation and is known to activate immune responses through Toll-like receptors. Its presence is indicative of ongoing inflammation, particularly in conditions like periodontitis. The observed decrease (Table 10) in calprotectin levels suggests that the treatment effectively reduced inflammatory activity in the oral cavity, contributing to improved oral health (Oktayoglu P, Mete N, Caglayan M, Bozkurt M, Bozan T, Em S, Nas K. Elevated serum levels of calprotectin (MRP8/MRP14) in patients with Behget's disease and its association with disease activity and quality of life. Scand J Clin Lab Invest. 2015 Apr;75(2):106-12)*.*

Finally, the role of Immunoglobulin A (IgA) in oral health cannot be overlooked. IgA plays a protective role against oral pathogens, and its levels are often reflective of the immune status of the oral cavity. The reduction in IgA levels observed (Table 10) post-treatment (1.66-fold, P=0.002154) could indicate a decrease in the need for immune defence, possibly due to the reduced inflammatory load and pathogen presence in the oral environment *(*Costalonga M, Herzberg MC. The oral microbiome and the immunobiology of periodontal disease and caries. Immunol Lett. 2014 Dec;162(2 Pt A):22-38)*.* As a final consideration, the elevated levels of N-acetylcadaverine and Spermidine indicate an alteration in polyamine metabolism, which is crucial for cell proliferation and the inflammatory response (Dimery IW, Nishioka K, Grossie VB Jr, Ota DM, Schantz SP, Byers R, Robbins KT, Hong WK. Polyamine metabolism in carcinoma of the oral cavity compared with adjacent and normal oral mucosa. Am J Surg. 1987 Oct;154(4):429-33.). This pathway is often dysfunctional in smokers, leading to the accumulation of inflammatory compounds and oral dysbiosis. The increase in 5-oxoproline suggests a greater demand for glutathione or other antioxidant molecules to counteract oxidative stress (Kumar J, Teoh SL, Das S, Mahakknaukrauh P. Oxidative Stress in Oral Diseases: Understanding Its Relation with Other Systemic Diseases. Front Physiol. 2017; 8:693. Published 2017 Sep 14)*.*

This pathway is important for maintaining redox balance in cells and protecting against oxidative damage induced by smoking. The increased levels of CXCL10, MCP-1, and NGAL in nicotine users highlight the activation of chemokine and cytokine pathways, which are essential for the recruitment of immune cells and modulation of the inflammatory response (Sahingur SE, Yeudall WA. Chemokine function in periodontal disease and oral cavity cancer. Front Immunol. 2015; 6:214. Published 2015 May 5)*.*

The metabolomic and proteomic data indicate that smokers experience significant elevations in pro-inflammatory markers, including a 14.12-fold increase in N-Acetylcadaverine and a 9.96-fold increase in MRP14, which are closely associated with oral dysbiosis and inflammation. Our findings demonstrate that supplementation with *Lactobacillus reuteri* and olive tree polyphenols can significantly mitigate these effects. The 1.77-fold reduction in N-Acetylcadaverine and the 1.84-fold reduction in CXCL10 following supplementation indicate a meaningful attenuation of the inflammatory state in smokers.

In general, these data indicate that a topical oral composition comprising *Lactobacillus reuteri* and olive tree polyphenols may be used as a medicament for pathological oral disorders which imply a mucosal inflammation and/or dysbiosis; therefore, this topical oral composition may be used as a medicament in one or more of the following pathological oral disorders: gingivitis, early or moderate stage periodontitis, oral candidiasis, chronic inflammation of the oral and/or oropharyngeal mucosa, inflammatory stomatitis, xerostomia, oral microbiota dysbiosis, chronic halitosis of microbial origin, mild *Streptococcus* pyogenes-induced pharyngitis or pharyngostomatitis.

Similarly, the composition may also be used in the treatment of one or more of the following non-pathological oral conditions: occasional (non-clinical) halitosis, oral irritation due to contact with nicotine-containing products, alteration of the oral microbial flora due to active or passive smoking; post-smoking-related oral dryness, prevention of dysbiosis and inflammation in current or former smokers, support for oral health during nicotine withdrawal, support for oral mucosal homeostasis under conditions of prolonged exposure to irritative agents such as tobacco and nicotine.

Moreover, data and evidences obtained using the herein disclosed composition may be extended also to any polyphenol or polyphenols produced by plants. As known, polyphenols are naturally occurring compounds of plant origin and include a diverse group of plant-derived compounds such as flavonoids (e.g., quercetin, catechins), phenolic acids (e.g., gallic acid), stilbenes (e.g., resveratrol), and lignans (e.g., secoisolariciresinol). Polyphenols not only directly influence bacterial cells but also impact proteins present in saliva and in the salivary pellicle. This leads to a 'tannin' effect, inhibiting bacterial adhesion by denaturing receptor proteins and affecting enzymes involved in bacterial metabolism. Rinsing with polyphenolic beverages has been shown to reduce the formation of bacterial glucans on tooth surfaces by decreasing bacterial adhesion sites. Furthermore, polyphenols enrich specific proteins in the pellicle layer, resulting in a thicker and denser pellicle that provides additional protection against erosion (Cardona F, Andrés-Lacueva C, Tulipani S, Tinahones FJ, Queipo-Ortuño MI. Benefits of polyphenols on gut microbiota and implications in human health. J Nutr Biochem. 2013 Aug;24(8):1415-22 -14)*.* Polyphenols are capable of damaging bacterial cell walls, interacting with intracellular membranes, and forming complexes with metal ions, thus reducing the availability of essential metals for microorganisms. They inhibit the adhesion of cariogenic bacteria to surfaces, glucan synthesis, and acid production. Moreover, polyphenols can modulate the defence mechanisms and metabolic processes of bacterial cells, ultimately influencing the structure, adhesion sites, and pathogenicity of biofilms (Satyanarayana M, Rajeswari KR. Biogenic amines in human gingiva in healthy and inflamed states. Indian J Dent Res. 1990 Apr-Sep;2(2-3):170-3*;* 15; Cheng J, Chen P, Song A, Wang D, Wang Q. Expanding lysine industry: industrial biomanufacturing of lysine and its derivatives. J Ind Microbiol Biotechnol. 2018 Aug;45(8):719-734).

In order to meet contingent and specific needs, a technician of the field can make numerous changes and modifications to the present invention in the embodiments shown and described, all thereby comprised in the protection scope of the invention as defined in the following claims.

## Claims

1. A topical oral composition comprising *Lactobacillus reuteri* and one or more polyphenols.

2. Topical oral composition according to claim 1, wherein said one or more polyphenols are olive tree polyphenols.

3. Topical oral composition according to claim 2, wherein the weight ratio of *Lactobacillus reuteri* to olive tree polyphenols ranges from 1:80 to 1:2.

4. Topical oral composition according to claims 2 or 3, wherein olive tree polyphenols comprise one or more of: hydroxytyrosol, tyrosol, Oleuropein.

5. Topical oral composition according to claims 1-4, wherein *Lactobacillus reuteri* is heat inactivated and/or tindalized.

6. A nicotine pouch comprising the topical oral composition according to claims 1-5.

7. A liquid formulation comprising the topical oral composition according to claims 1-5, wherein the formulation is suitable for administration by spray, drops, rinsing, or gargling.

8. A topical oral composition according to any of claims 1-5, for use as anti-inflammatory agent for oral disorders related to nicotine-containing products, or as agent for restoring microbial balance following chronic nicotine exposure, or for promoting and/or maintaining oral microbial homeostasis.

9. A topical oral composition according to any of claims 1-5, for use as medicament for oral disorders related to nicotine-containing products.

10. A topical oral composition according to any of claims 1-5, for use as medicament for one or more of the following pathological oral disorders: gingivitis, early or moderate stage periodontitis, oral candidiasis, chronic inflammation of the oral and/or oropharyngeal mucosa, inflammatory stomatitis, xerostomia, oral microbiota dysbiosis, chronic halitosis of microbial origin, mild *Streptococcus* pyogenes-induced pharyngitis or pharyngostomatitis.

11. Topical oral composition according to claims 9 or 10, wherein said topical oral composition comprises from 1 mg to 3 mg of *Lactobacillus reuteri* and from 10 mg to 80 mg of olive tree polyphenols.

12. Topical oral composition according to claims 9-11, wherein said topical oral composition is:
a) in liquid form and is administered by spray, drops, rinsing, or gargling; or
b) in solid form and is administered in the form of a pouch.

13. Topical oral composition according to claims 9-12, wherein said topical oral composition is administered once per day, twice per day or up to three times per day.

14. Topical oral composition according to claims 9-13, wherein *Lactobacillus reuteri* and said one or more polyphenols are administered separately or co-administered in a single formulation.

15. Topical oral composition according to claims 1-5, for use in the treatment of one or more of the following non-pathological oral conditions: occasional (non-clinical) halitosis, oral irritation due to contact with nicotine-containing products, alteration of the oral microbial flora due to active or passive smoking, post-smoking-related oral dryness, prevention of dysbiosis and inflammation in current or former smokers, support for oral health during nicotine withdrawal, support for oral mucosal homeostasis under conditions of prolonged exposure to irritative agents such as tobacco and nicotine.
